# EUROPÄISCHE PATENTANMELDUNG

(11) **EP 3 207 922 A1**
(43) Veröffentlichungstag der Anmeldung: **23.08.2017**
(21) Anmeldenummer: 17156611.0
(22) Anmeldetag: 17.02.2017
(51) Int. Cl.: A61K 9/20, A61K 9/28, A61K 31/4439

(54) **ORALE DARREICHUNGSFORM EINES DABIGATRAN-PRODRUGS ZUR BEHANDLUNG VON KRANKHEITEN**

(30) Priorität: 18.02.2016 DE 102016102887
(71) Anmelder: Develco Pharma Schweiz AG, 4133 Pratteln (CH)
(72) Erfinder: Clement, Bernd, 24161 Altenholz (DE)
(74) Vertreter: Ter Meer Steinmeister & Partner

(57) **Zusammenfassung**

Die Erfindung betrifft eine oral verabreichbare galenische Formulierung, umfassend
(i) mindestens einen Wirkstoff, der eine Verbindung der Formel 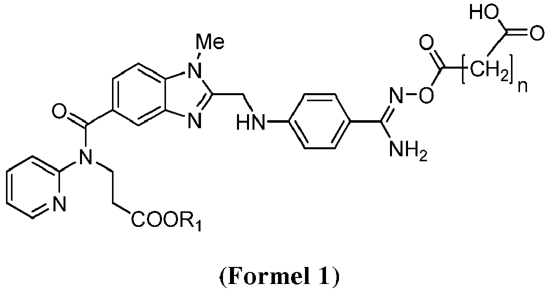 umfasst, in welcher
R¹
für ein -H, eine verzweigte oder unverzweigte, gesättigte oder ungesättigte, nicht substituierte Kohlenwasserstoffkette mit einer Kettenlänge von 1 bis 12 steht und
n
für 2 steht;
(ii) mindestens einen Füllstoff;
(iii) mindestens ein Bindemittel;
(iv) optional ein Fließregulierungsmittel;
(v) optional ein Sprengmittel;
(vi) optional ein Schmiermittel;
(vii) optional einen magensaftresistenten Überzug;
(viii) optional ein Retardierungsmittel.

## Beschreibung

Die vorliegende Erfindung betrifft oral verabreichbare galenische Formulierungen von Prodrug-Derivaten des Dabigatrans, ihre Verwendung zur Behandlung und/oder Prophylaxe von Krankheiten, insbesondere von thrombotischen Erkrankungen, von Schlaganfall, Herzinfarkt und/oder Vorhofflimmern und Arrhytmien, sowie von onkologischen Erkrankungen jeglicher Pathogenese.

Innerhalb der letzten Jahre hat sich Dabigatran Etexilat (Pradaxa®) in der Thrombose-Prophylaxe nach Hüft- und Kniegelenks-Operationen etabliert.¹ Zusätzlich wurde dieser Wirkstoff für weitere Indikationsgebiete (Vorhofflimmern, Schlaganfall-Prophylaxe und Sekundärprophylaxe nach Herzinfarkt, akutes Koronar-Syndrom) zugelassen.^{2, 3} Zudem sind auf lange Sicht weitere aussichtsreiche Indikationsgebiete denkbar, besonders im cardiovasculären Bereich sowie zur KrebsTherapie.⁴

Bei dem Wirkstoff Dabigatran handelt es sich um einen hochpotenten Thrombin-Inhibitor, der oral nicht verfügbar ist. Aus diesem Grund wird die Verbindung derzeit als Etexilat-Prodrug (Dabigatran-Etexilat, Pradaxa®) verwendet. Obwohl die Verbindung oral verfügbar und gut wirksam ist, besitzt die Verbindung aufgrund des verwendeten Prodrug-Prinzips erhebliche negative Eigenschaften. Die sehr schlechte Löslichkeit führt zu einigen Nachteilen sowohl bei der Anwendung als auch bei der Herstellung des Arzneimittels. Um die Löslichkeit der Verbindung zu verbessern wird der Arzneistoff auf Pellets, die Weinsäure enthalten, aufgetragen, was aufgrund eines erheblichen technischen Aufwands sehr kostenintensiv ist.⁵

Außerdem wird die Bioverfügbarkeit durch die schlechte Löslichkeit der Verbindung negativ beeinflusst, so dass die Medikation aus zwei Kapseln besteht, was wiederum die Compliance negativ beeinflusst.

Zwischenzeitlich ist es gelungen, verbesserte Dabigatran-Prodrugs in Form der Verbindungen der Formel 1 bereitzustellen: 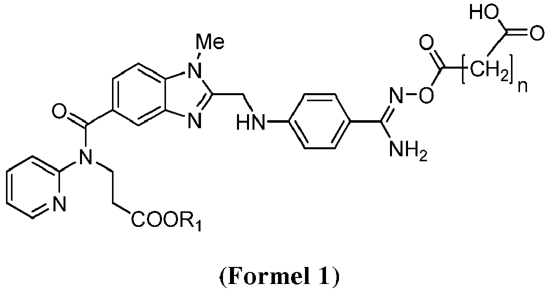 in welcher
- R¹: für ein -H, eine verzweigte oder unverzweigte, gesättigte oder ungesättigte, nicht substituierte Kohlenwasserstoffkette mit einer Kettenlänge von 1 bis 12 steht und
- n: für 2 steht.

Um das Potenzial dieses neuen Dabigatran-Prodrugs voll zu nutzen, ist es notwendig, eine galenische Formulierung des Wirkstoffes zu finden, die diese Eigenschaften voll zur Geltung bringt.

Vor diesem Hintergrund lag der vorliegenden Erfindung die Aufgabe zugrunde, eine oral verabreichbare galenische Formulierung von Dabigatran-Prodrugs der oben genannten Formel 1 bereitzustellen, die für die orale Verabreichung optimierte Eigenschaften aufweist. Dabei galt es insbesondere den Aspekt der Stabilität, auch bei Exposition der oral verabreichbaren galenischen Formulierungen gegenüber Luft, Sauerstoff und/oder Feuchtigkeit und/oder erhöhte Temperaturen zu gewährleisten und andererseits eine gleichbleibend hohe Bioverfügbarkeit, Magensaftresistenz und erwünschte Retardierungseigenschaften zu kombinieren.

Die genannte Aufgabe wird erfindungsgemäß gelöst durch eine oral verabreichbare galenische Formulierung, umfassend
(i) mindestens einen Wirkstoff, der eine Verbindung der Formel 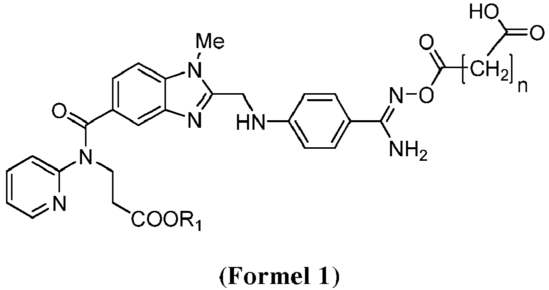 umfasst, in welcher
   - R¹: für ein -H, eine verzweigte oder unverzweigte, gesättigte oder ungesättigte, nicht substituierte Kohlenwasserstoffkette mit einer Kettenlänge von 1 bis 12 steht und
   - n: für 2 steht;
(ii) optional ein Füllstoff;
(iii) optional ein Bindemittel;
(iv) optional ein Fließregulierungsmittel;
(v) optional ein Sprengmittel;
(vi) optional ein Schmiermittel;
(vii) optional einen magensaftresistenten Überzug;
(viii) optional ein Retardierungsmittel.

Die genannte Aufgabe wird gemäß einer bevorzugten Ausführungsform gelöst durch eine oral verabreichbare galenische Formulierung, umfassend
(i) mindestens einen Wirkstoff, der eine Verbindung der Formel 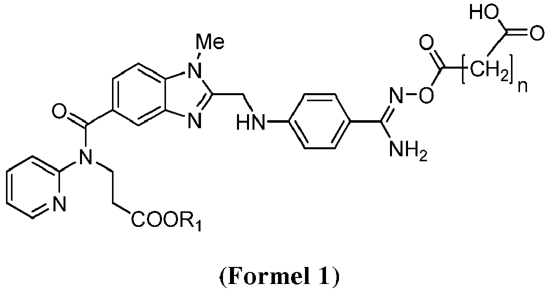 umfasst, in welcher
   - R¹: für ein -H, eine verzweigte oder unverzweigte, gesättigte oder ungesättigte, nicht substituierte Kohlenwasserstoffkette mit einer Kettenlänge von 1 bis 12 steht und
   - n: für 2 steht;
(ii) mindestens einen Füllstoff;
(iii) mindestens ein Bindemittel;
(iv) optional ein Fließregulierungsmittel;
(v) optional ein Sprengmittel;
(vi) optional ein Schmiermittel;
(vii) optional einen magensaftresistenten Überzug;
(viii) optional ein Retardierungsmittel.

Die genannte Aufgabe wird gemäß einer besonders bevorzugten Ausführungsform gelöst durch eine oral verabreichbare galenische Formulierung, umfassend
(i) mindestens einen Wirkstoff, der eine Verbindung der Formel 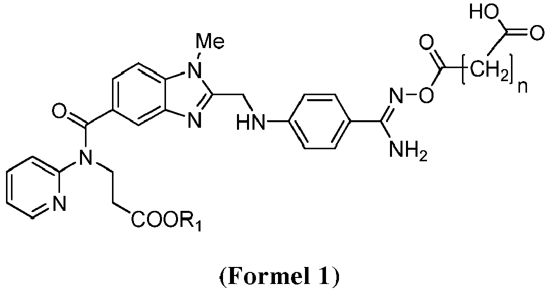 umfasst, in welcher
   - R¹: für ein -H, eine verzweigte oder unverzweigte, gesättigte oder ungesättigte, nicht substituierte Kohlenwasserstoffkette mit einer Kettenlänge von 1 bis 12 steht und
   - n: für 2 steht;
(ii) mindestens einen Füllstoff;
(iii) mindestens ein Bindemittel;
(iv) mindestens ein Fließregulierungsmittel;
(v) mindestens ein Sprengmittel;
(vi) mindestens ein Schmiermittel;
(vii) optional einen magensaftresistenten Überzug;
(viii) optional ein Retardierungsmittel.

Es hat sich gezeigt, dass durch diese oral verabreichbare galenische Formulierung des Dabigatran-Prodrugs eine hohe Stabilität erzielt wird und gleichzeitig optimierte Eigenschaften für die orale Einnahme gewährleistet sind.

In einer bevorzugten Ausführungsform ist die erfindungsgemäße galenische Formulierung so ausgestaltet, dass R¹ in Formel (1) für Ethyl steht.

In einer weiteren bevorzugten Ausführungsform ist die erfindungsgemäße galenische Formulierung so ausgestaltet, dass der Wirkstoff in Form von Salzen, Solvaten und Solvaten der Salze der Verbindung gemäß Formel (1) vorliegt.

In einer weiteren bevorzugten Ausführungsform ist die erfindungsgemäße galenische Formulierung so ausgestaltet, dass der Füllstoff (ii) ausgewählt ist aus der Gruppe, die aus Lactose Monohydrat, mikrokristalliner Zellulose und Mannitol besteht.

In einer weiteren bevorzugten Ausführungsform ist die erfindungsgemäße galenische Formulierung so ausgestaltet, dass das Bindemittel (iii) aus Calciummonohydrogenphosphat Dihydrat besteht.

In einer weiteren bevorzugten Ausführungsform ist die erfindungsgemäße galenische Formulierung so ausgestaltet, dass das Fließregulierungsmittel (iv) hochdisperses Siliciumdioxid ist.

In einer weiteren bevorzugten Ausführungsform ist die erfindungsgemäße galenische Formulierung so ausgestaltet, dass das Sprengmittel Maisstärke ist.

In einer weiteren bevorzugten Ausführungsform ist die erfindungsgemäße galenische Formulierung so ausgestaltet, dass das Schmiermittel (vi) Stearinsäure ist.

In einer weiteren bevorzugten Ausführungsform ist die erfindungsgemäße galenische Formulierung so ausgestaltet, dass der magensaftresistente Überzug (vii) Eudragit L ist.

In einer weiteren bevorzugten Ausführungsform ist die erfindungsgemäße galenische Formulierung so ausgestaltet, dass das Retardierungsmittel (viii) Eudragit RL ist.

In einer weiteren bevorzugten Ausführungsform ist die erfindungsgemäße galenische Formulierung so ausgestaltet, dass Povidon als Granuliermittel vorliegt.

In einer weiteren bevorzugten Ausführungsform ist die erfindungsgemäße galenische Formulierung so ausgestaltet, dass diese zudem einen weiteren Hilfsstoff in Form von Eisenoxid umfasst.

Es hat sich gezeigt, dass eine besonders vorteilhafte oral verabreichbare galenische Formulierung des Dabigatran-Prodrugs erzielt werden kann, wenn diese folgende Bestandteile umfasst:
(i) mindestens einen Wirkstoff, der eine Verbindung der Formel 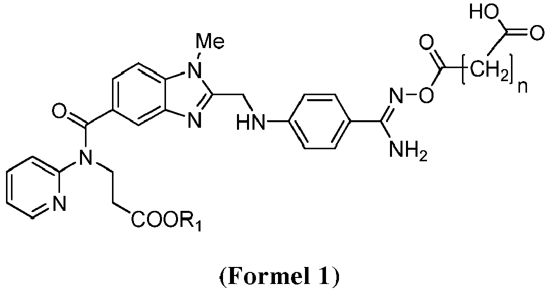 umfasst, in welcher
   - R¹: für ein -H, eine verzweigte oder unverzweigte, gesättigte oder ungesättigte, nicht substituierte Kohlenwasserstoffkette mit einer Kettenlänge von 1 bis 12 steht und
   - n: für 2 steht;
(ii) mindestens einen Füllstoff in Form von Mannitol;
(iii) mindestens ein Bindemittel in Form von Calciumphosphat Monohydrat;
(iv) mindestens ein Fließregulierungsmittel in Form von hochdispersem Siliciumdioxid;
(v) mindestens ein Sprengmittel in Form von Maisstärke;
(vi) mindestens ein Schmiermittel in Form von Stearinsäure;
(vii) optional ein Magensaftresistenzmittel in Form von Eudragit L 100;
(viii) optional ein Retardierungsmittel in Form von Eudragit RL PO.

Somit ist die erfindungsgemäße galenische Formulierung des Dabigatran-Prodrugs vorzugsweise in Form einer Tablette zur oralen Verabreichung, die eine verbesserte Stabilität des Wirkstoffes insbesondere bei Exposition gegenüber Luftsauerstoff und/oder Luftfeuchtigkeit aufweist.

Die vorliegende Erfindung betrifft insbesondere auch eine galenische Formulierung wie oben beschrieben zur Verbesserung der Stabilität eines Wirkstoffs gemäß der Formel 1.

### Material und Methoden: Ausführungsbeispiele

### Beispiel 1: Entwicklung einer Methode zur Probenvorbereitung für die Überprüfung der Presslingstabilität (NMR-Spektroskopie)

Von allen DAB-S-Hilfsstoff Presslinge und bei allen Bedingungen wurden nach 0 Tagen, 3 Tagen, 7 Tagen und 14 Tagen Proben gezogen. Im Falle von "open dish" wurden die Presslinge mit einer Pinzette aus dem Becherglas genommen und in ein Eppendorf Röhrchen (1.5 mL) transferiert. Die Presslinge, die in Alu-Alu Taschen bei 25°C und 40°C lagerten, wurden, um ein vollständiges Entnehmen zu gewährleisten, durch das Aufschneiden der Taschen in ein Eppendorf Röhrchen (1.5 mL) transferiert. Jeder Pressling im Röhrchen wurde vorsichtig mit einem Löffel zerkleinert und anschließend DMSO-*d*₆ (1000 µl) hinzugefügt. Die Suspension wurde für eine Minute geschüttelt und anschließend für 4 Minuten bei 13.000 rpm zentrifugiert, um alle unlöslichen Komponenten zu entfernen. Damit wurde eine vollständige Löslichkeit von DAB-S und DAB-AO gewährleistet. 500 µl des Überstandes wurden in ein NMR Röhrchen transferiert. Alle NMR-Proben wurden am Tag der Aufarbeitung gemessen. DAB-S ist in DMSO-*d*₆ über diesen Zeitraum stabil.

Um das Verhältnis von DAB-S zum Metaboliten DAB-AO bestimmen zu können, wurden die signifikanten Peaks im NMR-Spektrum analysiert. Zur Quantifizierung wurden die AUCs des Säure-OH Peaks (12.3 ppm) von DAB-S und der Amidoxim-OH Peak (9.3 ppm) von DAB-AO verwendet. Eine Zersetzung von DAB-S zu DAB-AO führt zu einer Reduzierung des DAB-S Peaks. Umgekehrt nimmt der DAB-AO Peak zu, sodass die Summe bei der AUCs auf 100% gesetzt werden konnte. Weitere Indikatoren für eine DAB-AO Bildung sind das Auftreten eines NH₂-Peaks bei 5.65 ppm sowie eine chemische Verschiebung der Signale für den aromatischen Ring bei 6.4 - 7.6 ppm.

Um die Auflösung der NMR-Spektren zu verbessern, wurde die Datenaufnahme für den Frequenzbereich von 5.5 - 14.5 ppm optimiert.

### Beispiel 2: Kompatibilität von Succinyl-Dabigatran in Presslingen mit Hilfsstoffen

### 2.1 Tabelle der Hilfsstoffe

Die exakten Produkte für alle Experimente sind in Tabelle 1 aufgelistet.

**Tabelle 1: Hilfsstoffe im dualen Gemisch mit DAB-S (2:1)**

| **Nr** | **Funktionalität** | **Hilfsstoff** | **Markenname** |
|---|---|---|---|
| **1** | Füllstoff | α-Lactose Monohydrat | Tablettose 80® |
| **2** | | MCC | Avicel® pH 101 |
| **3** | | Mannitol | Pearlitol® 300 DC |
| **4** | | Calciummonohydrogenphosphat-Dihydrat | Emcompress® |
| **5** | Fließregulierungsmittel | Aerosil® | Aerosil® 200 |
| **6** | Bindemittel | PVP 25 | Povidon® 25 |
| **7** | Zerfallsbeschleuniger | Maisstärke | Maisstärke |
| **8** | | Stearinsäure | Stearinsäure |
| | *Funktionelle Überzüge* | | |
| **9** | Magensaftresistent | anionisches Polymethylacrylat | Eudragit® L100 |
| **10** | Retardierung | Ammoniomethacrylat-Copolymer | Eudragit® RL PO |
| **11** | Andere | Eisenoxid | Sicovit® Braun 70 E172 |

### 2.2 Allgemeines

Die Presslinge wurden mit einer IR-Presse hergestellt und hatten ein Gewicht von 80 mg im Verhältnis Hilfsstoff zu DAB-S von 2 zu 1; bzw. von 53 mg zu 27 mg. An vier Zeitpunkten in zwei Wochen wurde ein DAB-S Pressling bei jeder Bedingung gemessen ("open dish", 25 °C Alu-Alu, 40 °C Alu-Alu): 0 Tage, 3 Tage, 7 Tage und 14 Tage. Die Vorbereitung der Presslinge startete Montags, Dienstags und Freitags um die Entnahme und unmittelbare Messungen zu gewährleisten und diese zu allen Zeitpunkten durchführen zu können. Drei Mischungen eines Hilfsstoffes mit DAB-S wurden pro Tag hergestellt, was zu 30 Presslingen an den jeweiligen Tagen führt.

Eine Referenzprobe aus reinem DAB-S der Charge, die zur Herstellung der Presslinge verwendet wurde, lagerte im Kühlschrank bei 7 °C über 14 Tage. Eine Entnahme erfolgte nach 0 Tagen und 14 Tagen.

### 2.3 Herstellung der Presslinge

572 mg der Stoffe und 286 mg DAB-S wurden für die IR-Presslinge in einer Reibschale zerrieben, bis eine homogene Mischung entstanden war. Exakt 80 mg wurden in ein Eppendorf Röhrchen (1.5 mL) transferiert. Der Inhalt jedes Röhrchens wurde in die IR- Presse gegeben und die Presslinge wurden mit einem Druck von 9 t über 2 Minuten gepresst.

Die NMR Analyse wurde direkt nach der Herstellung durchgeführt.

### 2.4 Lagerung der Presslinge

Die Presslinge wurden direkt nach der Herstellung unter den genannten Bedingungen gelagert (siehe Tabelle 2).

**Tabelle 2: Exakte Lagerungsbedingungen der dualen DAB-S-Hilfsmittel Pellets.**

| **Bedingung** | **Realisierung** |
|---|---|
| **"open dish"** | Die Presslinge wurden in einem offenen Becherglas im Labor ohne direkte Sonneneinstrahlung über 0 - 14 Tagen gelagert. |
| **25 °C Alu-Alu** | Die Presslinge wurden einzeln in einer verschließbaren Aluminiumtasche (12 µ Polyester/12 µ Aluminium/75 µ LDPE, Größe 6 × 10 cm, A 20T) in einer Klimakammer mit einer konstanten Temperatur von 25 °C über 0 - 14 Tage gelagert. |
| **40 °C Alu-Alu** | Die Presslinge wurden einzeln in einer verschließbaren Aluminiumtasche (12 µ Polyester/12 µ Aluminium/75 µ LDPE, Größe 6 × 10 cm, A 20T) in einer Klimakammer mit einer konstanten Temperatur von 40 °C über 0 - 14 Tage gelagert. |

### 2.5 Aufarbeitung für NMR-Analyse

Proben von allen DAB-S-Hilfsstoff Presslinge wurden bei allen Bedingungen nach 0 Tagen, 3 Tagen, 7 Tagen und 14 Tagen bearbeitet. Im Falle von "open dish" wurden die Presslinge mit einer Pinzette aus dem Becherglas genommen und in ein Eppendorf Röhrchen (1.5 mL) transferiert. Die Presslinge, die in Alu-Alu Taschen bei 25°C und 40°C lagerten, wurden, um ein vollständiges entnehmen zu gewährleisten, durch ein Aufschneiden der Taschen in ein Eppendorf Röhrchen (1.5 mL) transferiert. Jeder Pressling im Röhrchen wurde vorsichtig mit einer Pinzette zerkleinert und anschließend DMSO-*d*₆ (1000 µl) hinzugefügt. Die Suspension wurde 1 Minute geschüttelt und anschließend für 4 Minuten bei 13.000 rpm zentrifugiert, um alle unlöslichen Komponenten zu entfernen. So wurde eine vollständige Löslichkeit von DAB-S und DAB-AO gewährleistet. 500 µl des Überstandes wurde in ein NMR Röhrchen transferiert. Alle NMR Proben wurden am Tag der Aufarbeitung gemessen. DAB-S ist über diesen Zeitraum in DMSO-*d*₆ stabil.

### 2.6 NMR-Analyse

### 2.6.1 Allgemeines

Um das Verhältnis von DAB-S zum Metaboliten DAB-AO bestimmen zu können, wurden die signifikanten Peaks im NMR-Spektrum analysiert. Zur Quantifizierung wurden die AUCs des Säure-OH Peaks (12.3 ppm) von DAB-S und des Amidoxim-OH Peaks (9.3 ppm) von DAB-AO verwendet. Die Zersetzung von DAB-S zu DAB-AO führt zu einer Verringerung des DAB-S Peaks. Umgekehrt nimmt der DAB-AO Peak zu, sodass die Summe beider AUCs auf 100% gesetzt werden konnte. Weitere Indikatoren für eine DAB-AO Bildung sind das Auftreten eines NH₂-Peaks bei 5.65 ppm sowie eine chemische Verschiebung der Signale für den aromatischen Ring bei 6.4 - 7.6 ppm.

Um die Auflösung der NMR Spektren zu verbessern, wurde die Datenaufnahme für den Frequenzbereich von 5.5 - 14.5 ppm optimiert.

### 2.6.2 NMR Lösungsmittel

Mögliche NMR Lösungsmittel waren CDCl₃, DMSO-*d*₆, Aceton-*d*₆, CD₃OD und D₂O. Das hydrophile Lösungsmittel D₂O wurde aufgrund der bekannten Instabilität von DAB-S in wässrigen Lösungen ausgeschlossen. DAB-S zeigte sich unlöslich in CDCl₃, weshalb dieses als Lösungsmittel nicht geeignet war. In Aceton-*d*₆ und CD₃OD können die NH und OH Peaks teilweise mit Deuterium ausgetauscht werden, sodass die Intensität der genannten Signale verringert wird. In dieser Studie waren scharfe Peaks für die OH-Signale wichtig, sodass Aceton-*d*₆ und CD₃OD ebenfalls nicht geeignet waren. DAB-S ist löslich in DMSO-*d*₆ und die OH Peaks haben eine adäquate Auflösung, sodass DMSO-*d*₆ für alle NMR-Experimente verwendet wurde.

### 2.6.3 Vorversuch

NMR Spektren aller Hilfsmittel in DMSO-*d*₆ wurden mit der typischen Presslingmenge (80 mg/3*2 = 53 mg) gemessen um die Limitierung der Methode in Bezug auf Handhabung, Zunahme der Viskosität und Peak-Überlappung von DAB-S oder DAB-AO zu kontrollieren.

Ein Resultat ist, dass Aerosil® (5) im Gemisch mit DMSO-*d*₆ ein Gel bildet. Die Viskosität des Gels war zu hoch, um in ein NMR-Röhrchen transferiert zu werden.

Die NMR-Methode ist für die Hilfsmittel mit Säurefunktionen, Stearinsäure (15) und Eudragit® L100 (16) ungeeignet, da die Signale der Säure-OH Peaks im NMR-Spektrum mit den Signalen der OH-Gruppe des DAB-S bei 12.3 ppm überlappen.

**Tabelle 3: Herstellung der dualen DAB-S-Hilfsmittel Gemische und NMR-Messungen**

| **Action** |
|---|
| Vorbereitung der Presslinge 1, 2, 3 |
| NMR Messungen der Presslinge 1, 2, 3 nach 0 Tagen |
| NMR Messungen von reinem DAB-S (Referenz), 0 Tagen bei 7 °C |
| Vorbereitung der Presslinge 4, 6, 7 |
| NMR Messungen der Presslinge 4, 6, 7 nach 0 Tagen |
| NMR Messungen der Presslinge 1, 2, 3 nach 3 d "open dish", 25 °C Alu-Alu und 40 °C Alu-Alu |
| Vorbereitung der Presslinge 8, 9, |
| NMR Messungen der Presslinge 8, 9 nach 0 Tagen |
| NMR Messungen der Presslinge 4, 6, 7 nach 3 d "open dish", 25 °C Alu-Alu und 40 °C Alu-Alu |
| Vorbereitung der Presslinge 10, 11 |
| NMR Messung der Presslinge 10, 11 nach 0 Tagen |
| NMR Messungen der Presslinge 8, 9 nach 3 Tagen "open dish", 25 °C Alu-Alu und 40 °C Alu-Alu |
| NMR Messungen der Presslinge 1, 2, 3 nach 7 Tagen "open dish", 25 °C Alu-Alu und 40 °C Alu-Alu |
| NMR Messungen der Presslinge 4, 6, 7 nach 7 Tagen "open dish", 25 °C Alu-Alu und 40 °C Alu-Alu |
| NMR Messung der Presslinge 10, 11 nach 3 Tagen "open dish", 25°C Alu-Alu und 40°C Alu-Alu |
| NMR Messung der Presslinge 8,9 nach 7 Tagen "open dish", 25°C Alu-Alu und 40°C Alu-Alu |
| NMR Messungen der Presslinge 10,11 nach 7 Tagen "open dish", 25 °C Alu-Alu und 40 °C Alu-Alu |
| NMR Messungen der Presslinge 1, 2, 3 nach 14 Tagen "open dish", 25 °C Alu-Alu und 40 °C Alu-Alu |
| NMR Messung von reinem DAB-S (Referenz), 14 Tagen bei 7 °C |
| NMR Messungen der Presslinge 4, 6, 7 nach 14 Tagen "open dish", 25 °C Alu-Alu und 40 °C Alu-Alu |
| NMR Messungen der Presslinge 8, 9 nach 14 Tagen "open dish", 25 °C Alu-Alu und 40 °C Alu-Alu |
| NMR Messungen der Presslinge 10,11 nach 14 Tagen "open dish", 25 °C Alu-Alu und 40 °C Alu-Alu |

### 2.6.4 Ergebnisse

Um die Ergebnisse der Stabilitätstest der DAB-S-Ex Presslinge unter den genannten Bedingungen zu evaluieren, wurden die Säure-OH Peaks von DAB-S (12.3 ppm) und der Amidoxim-OH Peak von DAB-AO (9.3 ppm) ins Verhältnis zu der Summe aus beiden Peaks gesetzt um deren Menge in % zu erhalten. Variationen in den Werten sind auf teils breitere Peaks zurück zu führen, die durch die jeweiligen verwendeten Hilfsstoffe verursacht werden. Diese sind bis zu einer Abweichung von ± 2 % vom Anfangswert (0 Tage) nicht kritisch, insofern keine Tendenz erkennbar ist.

Um die Gültigkeit der Werte für die breiten Peaks zu bestätigen, wurde nach dem Auftreten des DAB-AO-NH₂ Peaks bei 5.6 ppm gesucht. (Der DAB-S-NH₂ Peak zeigt einen signifikanten chemischen Shift bei 6.5 ppm.) Das Bild der aromatischen Ringprotonen bei 6.3 bis 7.6 ppm wurde zusätzlich analysiert um die Bildung von DAB-AO zu detektieren.

In Tabelle 4 ist die Menge an DAB-S in den DAB-S-Ex Presslinge aufgelistet. Das Gegenstück dazu ist DAB-AO und wurde ebenfalls in den Spektren untersucht, insofern keine anderen Verbindungen erkennbar waren.

### Stabilität von reinem DAB-S im Kühlschrank über zwei Wochen

Das reine DAB-S zeigte sich über den Zeitraum von 2 Wochen im Kühlschrank bei 7 °C stabil. Nach 0 Tagen wurde eine Menge von 101 % DAB-S berechnet. Nach 14 Tagen ergab die Berechnung 100 % DAB-S. Es wurde kein DAB-AO-NH₂ Peak detektiert, ebenso keine typischen aromatischen DAB-AO Signale. Damit kann eine Bildung von DAB-AO aus DAB-S im Kühlschrank ausgeschlossen werden.

### 1. Tablettose® - Kompatibel

DAB-S war über den gesamten Zeitraum von zwei Wochen unter den Bedingungen "open dish", sowie bei 25 °C und 40 °C Umgebungstemperatur stabil, wenn es mit

Tablettose® (Lactose Monohydrat) gepresst wurde. Alle NMR Spektren waren eindeutig, sodass die Bildung von DAB-AO unter den getesteten Bedingungen ausgeschlossen werden kann (siehe Tabelle 4, Nummer 1).

### 2. Avicel® pH 101 - Kompatibel

DAB-S war über den gesamten Zeitraum von zwei Wochen unter den Bedingungen "open dish", sowie bei 25 °C und 40 °C Umgebungstemperatur stabil, wenn es mit Avicel® pH 101 (Mikrokristalline Zellulose) gepresst wurde. Alle NMR Spektren waren eindeutig, sodass eine Bildung von DAB-AO unter den getesteten Bedingungen ausgeschlossen werden kann (siehe Tabelle 4, Nummer 2).

### 3. Pearlitol® - Kompatibel

DAB-S war über den gesamten Zeitraum von zwei Wochen unter den Bedingungen "open dish", sowie bei 25 °C und 40 °C Umgebungstemperatur stabil, wenn es mit Pearlitol® (Mannitol) gepresst wurde. Alle NMR Spektren waren eindeutig, sodass eine Bildung von DAB-AO unter den getesteten Bedingungen ausgeschlossen werden kann (siehe Tabelle 4, Nummer 3).

### 4. Emcompress® - Kompatibel

DAB-S war über den gesamten Zeitraum von zwei Wochen unter den Bedingungen "open dish", sowie bei 25 °C und 40 °C Umgebungstemperatur stabil, wenn es mit Emcompress® (Calciummonohydrogenphosphat Dihydrat) gepresst wurde. Alle NMR Spektren waren eindeutig, sodass eine Bildung von DAB-AO unter den getesteten Bedingungen ausgeschlossen werden kann (siehe Tabelle 4, Nummer 4).

### 5. Aerosil® 200

Obwohl eine NMR-Messung aufgrund der Gelbildung nicht möglich war, gehen wir davon aus, dass Aerosil® 200 nicht zu Instabilitäten führt.

### 6. Povidon® 25 - Kompatibel

DAB-S-Povidon® (Polyvinylpyrrolidon) Presslinge verursachten in den NMR Spektren breite Peaks. Die bei 40 °C gelagerten Presslinge ergaben weniger breite Peaks im NMR im Vergleich zu den Presslinge, die unter den Bedingungen "open dish" gelagerten wurden. Die Evaluation der DAB-S und DAB-AO Mengen waren ungenau. Dennoch wurden keine DAB-AO-NH₂ Peaks und keine DAB-AO Signale im aromatischen Bereich detektiert, sodass angenommen werden kann, dass DAB-S stabil war. Die variierenden Werte nach 3 und 7 Tagen sind auf die breiten Peaks zurück zu führen, die nur ungenau zu integrieren sind. Nach 14 Tagen waren die Peaks weniger breit und es konnte ein Wert von 99 % für DAB-S festgestellt werden. Eine Rückbildung von DAB-AO zu DAB-S kann ausgeschlossen werden. Für die Bedingungen unter 25 °C und 40 °C kann die Stabilität von DAB-S ebenfalls angenommen werden, da die relevanten NMR-Peaks bei der 40 °C Probe scharf waren und somit eine Menge von 99 % DAB-S nach 14 Tagen bei 25 °C und 40 °C angenommen wird (siehe Tabelle 4, Nummer 6).

### 7. Maisstärke - Kompatibel

DAB-S war über den gesamten Zeitraum von zwei Wochen unter den Bedingungen "open dish", sowie bei 25 °C und 40 °C Umgebungstemperatur stabil, wenn es mit Maisstärke gepresst wurde. Alle NMR Spektren waren eindeutig, sodass eine Bildung von DAB-AO unter den getesteten Bedingungen ausgeschlossen werden kann (siehe Tabelle 4, Nummer 7).

### 8. Stearinsäure

Stearinsäure verfügt als Säure über saure OH-Protonen. In den NMR-Spektren überlappen die OH-Gruppen der Stearinsäure mit den sauren OH-Gruppen des DAB-S. Aus diesem Grund wurde der DAB-S Prozentanteil nicht berechnet und ist nicht wie für andere DAB-S-Ex Presslinge in Tabelle 4 angegeben. Dennoch wurden keine DAB-AO-NH₂ Peaks und keine DAB-AO Signale im aromatischen Bereich detektiert. DAB-S scheint stabil zu sein, wenn es mit Stearinsäure in einem Presslinge gepresst wird.

### 9. Eudragit®

Eudragit® L(Methacrylsäure - Methylmethacrylat Copolymer 1:1) enthält in seiner Struktur OH-Gruppen. In den NMR-Spektren überlappen die OH-Gruppen der Methacrylsäure mit den sauren OH des DAB-S. Aus diesem Grund wurde der DAB-S Prozentanteil nicht berechnet und ist nicht wie für andere DAB-S-Ex Pellets in Tabelle 4 angegeben. Dennoch wurden keine DAB-AO-NH₂ Peaks und keine DAB-AO Signale im aromatischen Bereich detektiert. DAB-S scheint stabil zu sein, wenn es mit Eudragit® L in einem Pressling gepresst wird.

### 10. Eudragit® RL- Kompatibel

Alle DAB-S-Eudragit® RL (Ammoniomethacrylat Copolymer, Typ A) Presslinge ergaben breite Peaks in den NMR-Spektren. Aus dem Grund waren die berechneten Prozentanteile von DAB-S und DAB-AO für das reale Verhältnis nicht repräsentativ. Dennoch wurden keine DAB-AO-NH₂ Peaks und keine DAB-AO Signale im aromatischen Bereich detektiert. Variierende Mengen an DAB-S werden auch hier durch die breiten Peaks und den daraus resultierenden ungenauen Integralen verursacht und sind kein Hinweis auf Zersetzung zu DAB-AO. Für alle Bedingungen kann angenommen werden, dass DAB-S stabil bleibt (Tabelle 4, Nummer 10).

### 11. Sicovit® E172 - Kompatibel

DAB-S war über den gesamten Zeitraum von zwei Wochen unter den Bedingungen "open dish", sowie bei 25 °C und 40 °C Umgebungstemperatur stabil, wenn es mit Sicovit® (Eisenoxid) gepresst wurde. Alle NMR-Spektren waren eindeutig, sodass eine Bildung von DAB-AO unter den getesteten Bedingungen ausgeschlossen werden kann (siehe Tabelle 4, Nummer 11).

**Tabelle 4: Menge von DAB-S (in %) als Ergebnis der NMR-Messungen der DAB-S-Ex Presslinge nach 0 - 14 Tagen**

| **Nr** | **Pressling DAB-S-Ex** | **Zeit** | **"open dish"** | **25 °C Alu-Alu** | **40 °C Alu-Alu** |
|---|---|---|---|---|---|
| **1** | Tablettose® | 0 Tage | 99 % | 99 % | 99 % |
| | | 3 Tage | 100 % | 100 % | 99 % |
| | | 7 Tage | 99 % | 99 % | 99 % |
| | | 14 Tage | 100 % | 99 % | 99 % |
| **2** | Avicel® pH 101 | 0 Tage | 99 % | 99 % | 99 % |
| | | 3 Tage | 99 % | 100 % | 100 % |
| | | 7 Tage | 101 % | 99 % | 100 % |
| | | 14 Tage | 97 % | 99 % | 100 % |
| **3** | Pearlitol® | 0 Tage | 101 % | 101 % | 101 % |
| | | 3 Tage | 101 % | 101 % | 101 % |
| | | 7 Tage | 102 % | 102 % | 102 % |
| | | 14 Tage | 102 % | 101 % | 101 % |
| **4** | Emcompress® | 0 Tage | 100 % | 100 % | 100 % |
| | | 3 Tage | 99 % | 100 % | 100 % |
| | | 7 Tage | 99 % | 99 % | 99 % |
| | | 14 Tage | 99 % | 99 % | 99 % |
| **5** | Aerosil® 200 | Aufgrund der Gelbildung waren mit Aerosil keine NMR-Messungen möglich. | | | |
| **6** | Povidon® 25 | 0 Tage | 97 % | 97 % | 97 % |
| | | 3 Tage | 80 % | 92 % | 99 % |
| | | 7 Tage | 94 % | 95 % | 99 % |
| | | 14 Tage | 99 % | 90 % | 99 % |
| **7** | Maisstärke | 0 Tage | 98 % | 98 % | 98 % |
| | | 3 Tage | 97 % | 98 % | 101 % |
| | | 7 Tage | 98 % | 98 % | 100 % |
| | | 14 Tage | 98 % | 98 % | 99 % |
| **8** | Stearinsäure | Aufgrund der Überlagerung von Signalen kann keine Quantifizierung vorgenommen werden. | | | |
| **9** | Eudragit® | Aufgrund der Überlagerung von Signalen kann keine Quantifizierung vorgenommen werden. | | | |
| **10** | Eudragit® RL | 0 Tage | 98 % | 98 % | 98 % |
| | | 3 Tage | 92 % | 95 % | 97 % |
| | | 7 Tage | 96 % | 97 % | 97 % |
| | | 14 Tage | 95 % | 97 % | 96 % |
| | | 14 Tage | 63 % | 48 % | 76 % |
| **11** | Sicovit® Braun 70 E172 | 0 Tage | 97 % | 97 % | 97 % |
| | | 3 Tage | 96 % | 96 % | 97 % |
| | | 7 Tage | 97 % | 97 % | 96% |
| | | 14 Tage | 96 % | 96 % | 97 % |

### 2.6.5 Diskussion

Die Ergebnisse demonstrieren die gute Eignung der Standard-Additions-NMR Methode für die vorliegenden Kompatibilitätsstudien. Die Bestimmung der Zersetzung von Succinyl-Dabigatran zu Dabigatran-Amidoxim in Presslinge konnten adäquat für die meisten aktiven Succinyl-Dabigatran/Hilfsstoff-Gemische durchgeführt werden.

Succinyl-Dabigatran zeigte sich in den Presslingen mit den meisten Hilfsstoffen stabil. In Verbindung mit allen Füllstoffen (Tablettose 80®, Avicel pH 101®, Pearlitol 300 DC® und Emcompress®) und unter allen Umgebungs- und Lagerungsbedingungen ("open dish", 25°C Alu-Alu; 40°C Alu-Alu) war es über einen Zeitraum von mindestens 14 Tagen stabil. Es konnte ebenfalls gezeigt werden, dass die Presslinge mit den getesteten funktionellen Zusatzstoffen, einschließlich magensaftresistenter Überzüge und Retardierungsmittel stabil waren. Der magensaftresistente Zusatzstoff Eudragit® L generierte Methacrylsäure-OH Peaks, die mit den Signalen überlappten, die bei den NMR-Messungen betrachtet wurden. Dennoch wurden keine typischen Dabigatran-Amidoxim Signale oder andere Derivate gefunden, sodass davon ausgegangen wird, dass Dabigatran-Amidoxim im Pressvorgang von Succinyl-Dabigatran mit Eudragit® L nicht gebildet wird und somit die Presslinge über einen Zeitraum von 14 Tagen stabil bleiben. Der Zusatzstoff Eudragit® RL PO, welcher für Retardatierungsformulierungen zusammen mit Succinyl-Dabigatran verwendet wurde, zeigte sich im Pressling für den Zeitraum von 14 Tagen unter allen Bedingungen stabil ("open dish", 25°C Alu-Alu; 40°C Alu-Alu). Der Flussregler Aerosil® ist ein gebräuchliches Geliermittel. Kompatibilitätsmessungen mit der NMR-Methode waren aufgrund der Gelbildung nicht möglich. Eine Zersetzung Hydrolyse von Succinyl-Dabigatran durch Aerosil® kann jedoch als sehr unwahrscheinlich betrachtet werden.

Bei zwei Succinyl-Dabigatran-Hilfsstoff Gemischen (Stearinsäure und Eudragit®) konnte die Zersetzung von Succinyl-Dabigatran zu Dabigatran-Amidoxim nicht gänzlich ausgeschlossen werden, da in diesen Fällen die substanzspezifischen NMR-Signale im gleichen Bereich liegen wie die Signale, die zur Quantifizierung von Dabigatran-Amidoxim verwendet werden. Dennoch zeigen die NMR-Daten, dass bei Stearinsäure und Eudragit L die Bildung von Dabigatran-Amidoxim eher unwahrscheinlich ist.

Zusammengefasst zeigen die hier durchgeführten Studien, dass Succinyl-Dabigatran für den herkömmlichen Pressvorgang geeignet ist. Da der aktive Inhaltsstoff in Presslingen, Tabletten oder Pillen hauptsächlich von Füllstoffen umgeben ist, wird ausgehend von den vorliegenden exzellenten Ergebnissen der Stabilitätstests in den Succinyl-Dabigatran/Füllstoff-Presslingen keine Zersetzung zu Dabigatran-Amidoxim erwartet. Succinyl-Dabigatran/Eudgragit® L100 Presslinge, ebenso wie Succinyl-Dabigatran/Eudragit® RL PO Presslinge zeigen keine Bildung von Dabigatran-Amidoxim und demonstrieren so die mögliche Verwendung für magensaftresistente Überzüge sowie Retardierungsformulierungen im Allgemeinen.

Die folgende Formulierung zur direkten Kompression hat sich als besonders vorteilhaft erwiesen:

| | |
|---|---|
| Aktiver Wirkstoff: | Succinyl-Dabigatran |
| Füllmaterial: | Pearlitol® (Mannitol) |
| Trockenes Bindemittel: | Emcompress® (Calciummonohydrogenphosphat Dihydrat) |
| Fließregulierungsmittel: | Aerosil® 200 |
| Sprengmittel: | Maisstärke |
| Schmiermittel: | Stearinsäure |
| Magensaftresistente Überzüge: | Eudragit L |
| Retardierung: | Eudragit RL PO |

### Literatur

1. Lassen, MR. Recent developments in the use of oral anticoagulants. Expert Opin Pharmacother 2009, 10,1769-81
2. Stangier, J.; Stahle, H.; Rbeihgen, K.; Furh, R.; Pharmacokinetics and pharmacodynamics of the direct thrombin inhibitor Dabugatran in healthy elderly subjects. Clin. Pharmacokinet 2008, 47, 47-49
3. Sanford, M.; Plosker, G.L. Dabigatran Etexilate. Drugs 2008, 51, 8173-7
4. Peacock, W.F.; Levy, P.D.; Gonzalez, M.G.; Than, M. Target specific oral anticoagulants in the emergency department. The journal of emergency medicine 2015
5. http://www.ema.europa.eu/docs/de_DE/document_library/EPAR_-Product_Information/human/000829/WC500041059.pdf

## Patentansprüche

1. Oral verabreichbare galenische Formulierung, umfassend
(i) mindestens einen Wirkstoff, der eine Verbindung der Formel 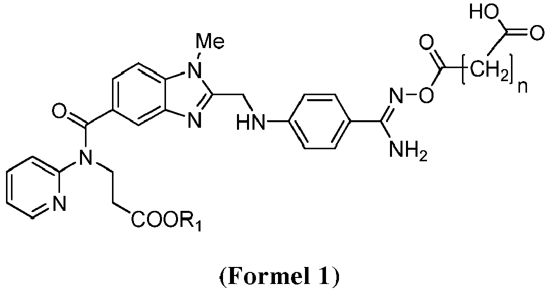 umfasst, in welcher
R¹ für ein -H, eine verzweigte oder unverzweigte, gesättigte oder ungesättigte, nicht substituierte Kohlenwasserstoffkette mit einer Kettenlänge von 1 bis 12 steht und
n für 2 steht;
(ii) mindestens einen Füllstoff;
(iii) mindestens ein Bindemittel;
(iv) optional ein Fließregulierungsmittel;
(v) optional ein Sprengmittel;
(vi) optional ein Schmiermittel;
(vii) optional einen magensaftresistenten Überzug;
(viii) optional ein Retardierungsmittel.

2. Galenische Formulierung nach Anspruch 1, wobei R¹ in Formel (1) für Ethyl steht.

3. Galenische Formulierung nach Anspruch 1 oder 2, wobei der Wirkstoff in Form von Salzen, Solvaten und Solvaten der Salze der Verbindung gemäß Formel (1) vorliegt.

4. Galenische Formulierung gemäß mindestens einem der vorhergehenden Ansprüche, worin der Füllstoff (ii) ausgewählt ist aus der Gruppe, die aus Lactose Monohydrat, Lactose Anhydrat, mikrokristalliner Zellulose und Mannitol besteht.

5. Galenische Formulierung gemäß mindestens einem der vorhergehenden Ansprüche, worin das Bindemittel (iii) aus Calciummonohydrogenphosphat Dihydrat besteht.

6. Galenische Formulierung gemäß mindestens einem der vorhergehenden Ansprüche, wobei das Fließregulierungsmittel (iv) hochdisperses Siliciumdioxid ist.

7. Galenische Formulierung gemäß mindestens einem der vorhergehenden Ansprüche, wobei das Sprengmittel Maisstärke ist.

8. Galenische Formulierung gemäß mindestens einem der vorhergehenden Ansprüche, wobei das Schmiermittel (vi) Stearinsäure ist.

9. Galenische Formulierung gemäß mindestens einem der vorhergehenden Ansprüche, wobei der magensaftresistente Überzug (vii) Eudragit L ist.

10. Galenische Formulierung gemäß mindestens einem der vorhergehenden Ansprüche, wobei das Retardierungsmittel (viii) Eudragit RL oder Eudragit RL PO ist.

11. Galenische Formulierung gemäß mindestens einem der vorhergehenden Ansprüche, bei der zudem Povidon Granuliermittel ist.

12. Galenische Formulierung gemäß mindestens einem der vorhergehenden Ansprüche, die zudem einen weiteren Hilfsstoff in Form von Eisenoxid umfasst.

13. Oral verabreichbare galenische Formulierung gemäß einem der vorhergehenden Ansprüche, umfassend
(i) mindestens einen Wirkstoff, der eine Verbindung der Formel 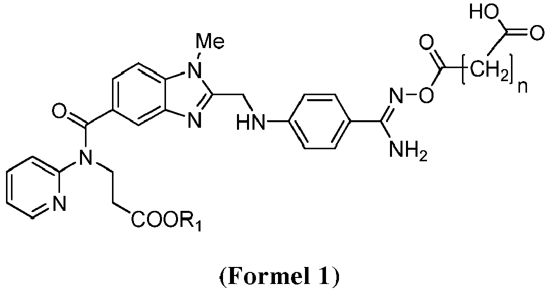 umfasst, in welcher
R¹ für ein -H, eine verzweigte oder unverzweigte, gesättigte oder ungesättigte, nicht substituierte Kohlenwasserstoffkette mit einer Kettenlänge von 1 bis 12 steht und
n für 2 steht;
(ii) mindestens einen Füllstoff in Form von Mannitol;
(iii) mindestens ein Bindemittel in Form von Calciummonohydrogenphosphat Dihydrat;
(iv) mindestens ein Fließregulierungsmittel in Form von hochdispersem Siliciumdioxid;
(v) mindestens ein Sprengmittel in Form von Maisstärke;
(vi) mindestens ein Schmiermittel in Form von Stearinsäure;
(vii) optional ein Magensaftresistenzmittel in Form von Eudragit L;
(viii) optional ein Retardierungsmittel in Form von Eudragit RL PO.

14. Galenische Formulierung gemäß mindestens einem der vorhergehenden Ansprüche in Form einer Tablette zur oralen Verabreichung, die eine verbesserte Stabilität des Wirkstoffes insbesondere bei Exposition gegenüber Luftsauerstoff und/oder Luftfeuchtigkeit aufweist.

15. Galenische Formulierung gemäß einem der Ansprüche 1-12 zur Verbesserung der Stabilität eines Wirkstoffs gemäß der Formel 1.
